# EUROPEAN PATENT APPLICATION

(11) **EP 2 050 449 A1**
(43) Date of publication of application: **22.04.2009**
(21) Application number: 07791601.3
(22) Date of filing: 30.07.2007
(51) Int. Cl.: A61K 31/437, A61K 9/08, A61K 47/12, A61K 47/18, A61K 47/36, A61P 27/06, A61P 43/00, C07D 487/04

(54) **AQUEOUS LIQUID PREPARATION CONTAINING AMIDE COMPOUND**

(30) Priority: 31.07.2006 JP 2006209102
(71) Applicant: Senju Pharmaceutical Co., Ltd., Osaka-shi, Osaka 541-0046 (JP); Mitsubishi Tanabe Pharma Corporation, Chuo-ku Osaka-shi Osaka 541-8505 (JP)
(72) Inventor: SAWA, Shirou, Kobe-shi Hyogo 651-2241 (JP); FUJII, Hiroyuki, Osaka-shi Osaka 541-0046 (JP)
(74) Representative: Weber, Thomas
(86) International application number: PCT/JP2007/064916
(87) International publication number: WO 2008/016016

(57) **Abstract**

An object of the present invention is to promote penetration of a compound having a Rho kinase inhibitory activity to a topical moiety, and provide an aqueous liquid preparation capable of maintaining a given drug concentration even when administration frequency is reduced. The present invention solves the aforementioned problem by an aqueous liquid preparation containing a compound represented by the following formula (I) 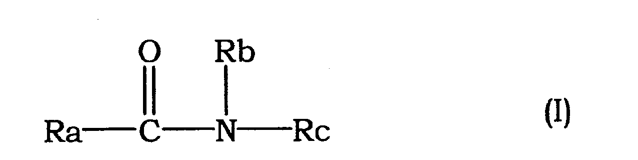 wherein each symbol is as defined in the specification, an isomer thereof and/or a pharmaceutically acceptable acid addition salt thereof, and an ion-pairing reagent or a water-soluble polymer.

## Description

### Technical Field

The present invention relates to an aqueous liquid preparation containing an amide compound. More particularly, the present invention relates to an ophthalmic preparation containing an amide compound having a Rho kinase inhibitory activity as an active ingredient, which promotes intraocular penetration of the active ingredient.

### Background Art

Glaucoma is caused by an abnormally high internal pressure of the eyeball, wherein the abnormally high pressure makes the eye grow dim or hurts the eye, which in turn fails the eyesight little by little possibly into blindness. Normally, hydatoid continuously circulates in the eyeball and maintains a constant intraocular pressure (10 - 20 mmHg). The pressure is maintained by the circulation of the blood and lymphocytes, elasticity of the eyeball wall, the performance of the control nerves and the like. An abnormality in any of them results in a rise of the intraocular pressure, which may develop glaucoma.

With the aim of preventing the intraocular pressure from rising or lowering an intraocular pressure that went up, for the prophylaxis and treatment of glaucoma, various drugs have been used. Known eye drops for the therapy of glaucoma include sympathetic agonists such as epinephrine, dipivefrine and the like. In addition, β-adrenaline blockers such as timolol, pindolol and the like are widely used since they advantageously decrease intraocular pressure by suppressing production of aqueous humor, and do not act on the pupil. A recent suggestion of an aqueous humor outflow promoting effect of α1-adrenalin blockers also suggests potential use of bunazosin hydrochloride and the like as a new therapeutic agent of glaucoma (non-patent reference 1).

In the meantime, a compound having a Rho kinase inhibitory activity has been reported to show a hypotensive effect on various hypertension model animals (non-patent reference 2). The Rho kinase has been confirmed to be present in corneal epithelial cells (non-patent reference 3).

As a compound having a Rho kinase inhibitory activity, a compound of formula (I) to be mentioned later has been reported (patent reference 1). The compound described in patent reference 1 is known to be useful as an agent for the prophylaxis and treatment of disorders of circulatory organs such as coronary, cerebral, renal, peripheral artery and the like (e.g., a therapeutic agent of hypertension, a therapeutic agent of angina pectoris, a therapeutic agent of renal and peripheral circulation disorder, a suppressive agent of cerebrovascular contraction and the like), a therapeutic agent of asthma, which are potent and long lasting, and further in the ophthalmology area, useful for retinopathy. In addition, patent reference 2 discloses that the compound of the formula (I) is useful for the prophylaxis or treatment of glaucoma, patent reference 3 discloses that the compound is useful for the treatment of vision dysfunction, and patent reference 4 discloses that the compound is useful for the recovery of corneal sensitivity.

As mentioned above, various components effective for the prophylaxis or treatment of glaucoma are known. It is important to promote penetration thereof to aqueous humor in order to sustain the efficacy. For example, patent reference 5 discloses addition of straight chain fatty acid having a carbon number of 6 to 10, alkylsulfonic acid, phosphoric acid or citric acid having a carbon number of 6 to 10, or a salt thereof, to enhance the cornea permeability of bunazosin or brazosin. Patent reference 6 discloses use of C3 - C7 fatty acid or a salt thereof to promote the cornea permeability of a β blocker (carteolol, timolol etc.). Non-patent reference 4 discloses instillation of ion-pair of timolol and caprylic acid to a rabbit to increase the amount of timolol penetrated into the aqueous humor. Moreover, patent reference 7 discloses an ophthalmologic composition containing xanthan gum and a carbonate dehydratase inhibitor for the treatment of glaucoma. Patent reference 8 discloses an ophthalmic liquid composition which becomes gel on instillation to the eye, has total ion intensity of not more than 120 mM, contains particular xanthan gum and is free of locust bean gum. As a drug to be contained in the composition, an anti-glaucoma agent (timolol, brimonidine, latanoprost etc.) is described therein.
patent reference 1: WO98/06433
patent reference 2: WO00/09162
patent reference 3: WO02/083175
patent reference 4: WO2005/118582
patent reference 5: JP-A-63-301822
patent reference 6: WO99/22715
patent reference 7: JP-A-2001-508035
patent reference 8: JP-A-2002-510654
non-patent reference 1: Folia Ophthalmologica Japonica, vol. 42, pp. 710 - 714, 1990
non-patent reference 2: Masayoshi Uehata, et al., Nature 389, 990-994, 1997
non-patent reference 3: Nirmala SundarRaj, et al., IOVS, 39(7) 1266-1272, 1998
non-patent reference 4: J. Pharm. Biomed. Anal., Vol.7, No.4, pp. 433-439, 1989

### Disclosure of the Invention

### Problems to be Solved by the Invention

An object of the present invention is to promote penetration of a compound having a Rho kinase inhibitory activity to a topical moiety, and provide an aqueous liquid preparation capable of maintaining a given drug concentration even when administration frequency is reduced.

### Means of Solving the Problems

The present inventors have conducted intensive studies in view of the above-mentioned problems and found a blending component which promotes topical penetration of a compound having a Rho kinase inhibitory activity, which resulted in the completion of the present invention. Accordingly, the present invention relates to the following.

(1) An aqueous liquid preparation comprising an amide compound represented by the following formula (I)

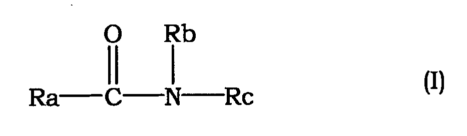

wherein
- Ra: is a group of the formula

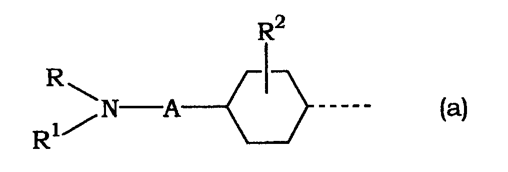

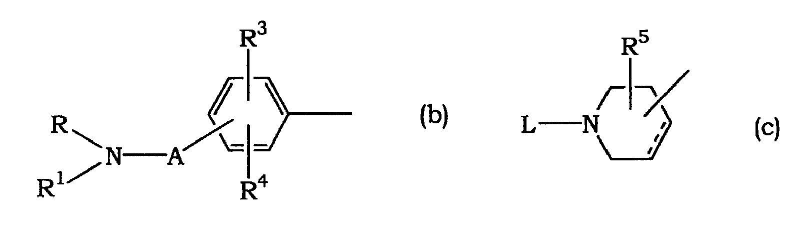

in the formulas (a) and (b),
- R: is hydrogen, alkyl, or cycloalkyl, cycloalkylalkyl, phenyl or aralkyl, which optionally has a substituent on the ring, or a group of the formula

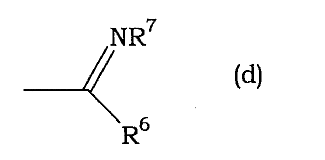

wherein R⁶ is hydrogen, alkyl or formula: -NR⁸R⁹ wherein R⁸ and R⁹ are the same or different and each is hydrogen, alkyl, aralkyl or phenyl, R⁷ is hydrogen, alkyl, aralkyl, phenyl, nitro or cyano, or R⁶ and R⁷ in combination show a group forming a heterocycle optionally further having, in the ring, oxygen atom, sulfur atom or optionally substituted nitrogen atom,
- R¹: is hydrogen, alkyl, or cycloalkyl, cycloalkylalkyl, phenyl or aralkyl, which optionally has a substituent on the ring,
- or R and R¹: in combination form, together with the adjacent nitrogen atom, a group forming a heterocycle optionally further having, in the ring, oxygen atom, sulfur atom or optionally substituted nitrogen atom,
- R²: is hydrogen or alkyl,
- R³ and R⁴: are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and
- A: is a group of the formula

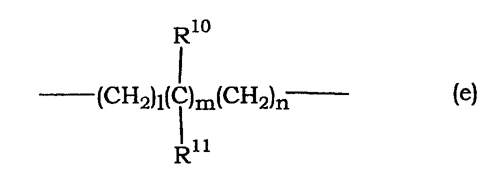

wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
in the formula (c),
- L: is hydrogen, alkyl, aminoalkyl, mono- or dialkylaminoalkyl, tetrahydrofurfuryl, carbamoylalkyl, phthalimidoalkyl, amidino or a group of the formula

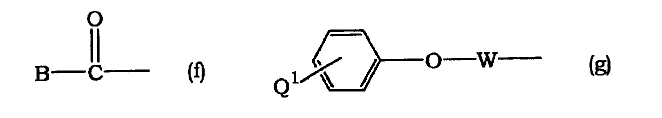

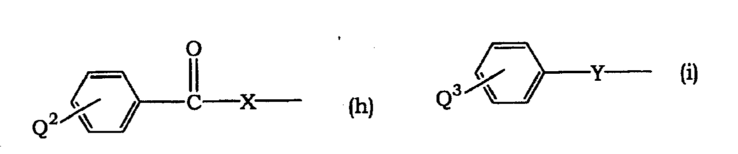

wherein B is hydrogen, alkyl, alkoxy, aralkyl, aralkyloxy, aminoalkyl, hydroxyalkyl, alkanoyloxyalkyl, alkoxycarbonylalkyl, α-aminobenzyl, furyl, pyridyl, phenyl, phenylamino, styryl or imidazopyridyl,
Q¹ is hydrogen, halogen, hydroxy, aralkyloxy or thienylmethyl,
W is alkylene,
Q² is hydrogen, halogen, hydroxy or aralkyloxy,
X is alkylene,
Q³ is hydrogen, halogen, hydroxy, alkoxy, nitro, amino, 2,3-dihydrofuryl or 5-methyl-3-oxo-
2,3,4,5-tetrahydropyridazin-6-yl;
and Y is a single bond, alkylene or alkenylene, and
in the formula (c),
a broken line is a single bond or a double bond, and
- R⁵: is hydrogen, hydroxy, alkoxy, alkoxycarbonyloxy, alkanoyloxy or aralkyloxycarbonyloxy;
- Rb: is hydrogen, alkyl, aralkyl, aminoalkyl or mono- or dialkylaminoalkyl; and
- Rc: is an optionally substituted heterocycle containing nitrogen,
an isomer thereof and/or a pharmaceutically acceptable acid addition salt thereof (hereinafter sometimes to be referred to as the present compound), and an ion-pairing reagent or water-soluble polymer.
(2) The aqueous liquid preparation of the aforementioned (1), wherein the ion-pairing reagent is sorbic acid, octanoic acid or N-lauroyl-L-glutamic acid or a pharmacologically acceptable salt thereof.
(3) The aqueous liquid preparation of the aforementioned (1), wherein the water-soluble polymer is xanthan gum.
(4) The aqueous liquid preparation of any one of the aforementioned (1) - (3), wherein the compound represented by the formula (I) is (R)-(+)-N-(1H-pyrrolo[2,3-b]pyridin-4-yl)-4-(1-aminoethyl)benzamide·monohydrochloride.
(5) The aqueous liquid preparation of any one of the aforementioned (1) - (4), which is an eye drop.
(6) A method of promoting intraocular penetration of a compound represented by the formula (I) of the aforementioned (1), an isomer thereof and/or a pharmaceutically acceptable acid addition salt thereof, which comprises adding an ion-pairing reagent or a water-soluble polymer to an aqueous solution comprising the compound represented by the formula (I), or an isomer thereof and/or a pharmaceutically acceptable acid addition salt thereof.
(7) A method for the prophylaxis or treatment of a disease selected from the group consisting of glaucoma, asthenopia and pseudomyopia caused by sustained abnormal tension of ciliary muscle, a visual functional disorder caused by damage or degeneration of retinal nerve or optic nerve, corneal sensitivity dysfunction caused by damage to corneal nerve and dry eye caused by corneal sensitivity dysfunction, which comprises administering an effective amount of the compound represented by the formula (I) of the aforementioned (1), an isomer thereof and/or a pharmaceutically acceptable acid addition salt thereof to a subject together with an ion-pairing reagent or a water-soluble polymer.
(8) The method of the aforementioned (7), wherein the ion-pairing reagent is sorbic acid, octanoic acid or N-lauroyl-L-glutamic acid or a pharmacologically acceptable salt thereof.
(9) The method of the aforementioned (7), wherein the water-soluble polymer is xanthan gum.
(10) The method of any one of the aforementioned (7) - (9), wherein the compound represented by the formula (I) is (R)-(+)-N-(1H-pyrrolo[2,3-b]pyridin-4-yl)-4-(1-aminoethyl)benzamide·monohydrochloride.
(11) The method of any one of the aforementioned (7) - (10), which relates to an eye drop.
(12) Use of the compound represented by the formula (I) of the aforementioned (1), an isomer thereof and/or a pharmaceutically acceptable acid addition salt thereof, and an ion-pairing reagent or a water-soluble polymer for the production of a water-soluble liquid preparation for the prophylaxis or treatment of a disease selected from the group consisting of glaucoma, asthenopia and pseudomyopia caused by sustained abnormal tension of ciliary muscle, a visual functional disorder caused by damage or degeneration of retinal nerve or optic nerve, corneal sensitivity dysfunction caused by damage to corneal nerve and dry eye caused by corneal sensitivity dysfunction.
(13) The use of the aforementioned (12), wherein the ion-pairing reagent is sorbic acid, octanoic acid or N-lauroyl-L-glutamic acid or a pharmacologically acceptable salt thereof.
(14) The use of the aforementioned (12), wherein the water-soluble polymer is xanthan gum.
(15) The use of any one of the aforementioned (12) - (14), wherein the compound represented by the formula (I) is (R)-(+)-N-(1H-pyrrolo[2,3-b]pyridin-4-yl)-4-(1-aminoethyl)benzamide·monohydrochloride.
(16) The use of any one of the aforementioned (12) - (15), which relates to an eye drop.
(17) A commercial package comprising the aqueous liquid preparation of any one of the aforementioned (1) - (5) and a written matter stating that the aqueous liquid preparation can or should be used for the prophylaxis or treatment of a disease selected from the group consisting of glaucoma, asthenopia and pseudomyopia caused by sustained abnormal tension of ciliary muscle, a visual functional disorder caused by damage or degeneration of retinal nerve or optic nerve, corneal sensitivity dysfunction caused by damage to corneal nerve and dry eye caused by corneal sensitivity dysfunction.

### Effect of the Invention

According to the aqueous liquid preparation of the present invention, intraocular penetration of the present compound which is an active ingredient is promoted, a treatment-effective concentration of the active ingredient can be maintained for a long time in an intraocular tissue, and the administration frequency can be reduced. According to the present invention, a preparation superior in the action site reacheability of the active ingredient, effective use of the ingredient and reduction of administration frequency, as compared to known preparations containing the same concentration of the active ingredient, can be provided. According to the present invention, moreover, even a preparation containing a low concentration of the active ingredient can exhibit efficacy equivalent to that of known preparations, and extend the administration interval. Thus, a preparation less burdensome particularly to patients under long-term administration can be provided.

### Brief Description of the Drawings

Fig. 1 is a graph showing the concentration of compound A in aqueous humor after instillation administration.
Fig. 2 is a graph showing the concentration of compound A in conjunctiva after instillation administration.

### Best Mode for Carrying Out the Invention

In the present invention, Rho kinase means serine/threonine kinase activated along with the activation of Rho. For example, ROKα (ROCKII: Leung, T. et al, J. Biol. Chem., 270, 29051-29054, 1995), p160 ROCK (ROKβ, ROCK-I: Ishizaki, T. et al, The EMBO J., 15(8), 1885-1893, 1996) and other proteins having a serine/threonine kinase activity are exemplified.

In the present invention, one kind of a compound having a Rho kinase inhibitory activity may be used alone or, where necessary, several kinds thereof may be used concurrently.

In the present specification, each symbol of the formula (I) is defined as follows.
Alkyl at R and R¹ is linear or branched alkyl having 1 to 10 carbon atoms, which is exemplified by methyl, ethyl, propyl, isopropyl, butyl, isobutyl, sec-butyl, tert-butyl, pentyl, hexyl, heptyl, octyl, nonyl, decyl and the like, with preference given to alkyl having 1 to 4 carbon atoms.
Cycloalkyl at R and R¹ has 3 to 7 carbon atoms and is exemplified by cyclopropyl, cyclobutyl, cyclopentyl, cyclohexyl, cycloheptyl and the like.
Cycloalkylalkyl at R and R¹ is that wherein the cycloalkyl moiety is the above-mentioned cycloalkyl having 3 to 7 carbon atoms and the alkyl moiety is linear or branched alkyl having 1 to 6 carbon atoms (methyl, ethyl, propyl, isopropyl, butyl, pentyl, hexyl etc.), and cyclopropylmethyl, cyclobutylmethyl, cyclopentylmethyl, cyclohexylmethyl, cycloheptylmethyl, cyclopropylethyl, cyclopentylethyl, cyclohexylethyl, cycloheptylethyl, cyclopropylpropyl, cyclopentylpropyl, cyclohexylpropyl, cycloheptylpropyl, cyclopropylbutyl, cyclopentylbutyl, cyclohexylbutyl, cycloheptylbutyl, cyclopropylhexyl, cyclopentylhexyl, cyclohexylhexyl, cycloheptylhexyl and the like can be mentioned.

Aralkyl at R and R¹ is that wherein alkyl moiety is alkyl having 1 to 4 carbon atoms and is exemplified by phenylalkyl such as benzyl, 1-phenylethyl, 2-phenylethyl, 3-phenylpropyl, 4-phenylbutyl and the like.
The substituent of optionally substituted cycloalkyl, cycloalkylalkyl, phenyl and aralkyl on the ring at R and R¹ is halogen (e.g., chlorine, bromine, fluorine and iodine), alkyl (same as alkyl at R and R¹), alkoxy (linear or branched alkoxy having 1 to 6 carbon atoms, such as methoxy, ethoxy, propoxy, isopropoxy, butoxy, isobutoxy, sec-butoxy, tert-butoxy, pentyloxy, hexyloxy and the like), aralkyl (same as aralkyl at R and R¹) or haloalkyl (alkyl at R and R¹ which is substituted by 1-5 halogen, and exemplified by fluoromethyl, difluoromethyl, trifluoromethyl, 2,2,2-trifluoroethyl, 2,2,3,3,3-pentafluoropropyl and the like), nitro, amino, cyano, azide and the like.
The group formed by R and R¹ in combination together with the adjacent nitrogen atom, which forms a heterocycle optionally further having, in the ring, oxygen atom, sulfur atom or optionally substituted nitrogen atom is preferably a 5 or 6-membered ring and bonded ring thereof. Examples thereof include 1-pyrrolidinyl, piperidino, 1-piperazinyl, morpholino, thiomorpholino, 1-imidazolyl, 2,3-dihydrothiazol-3-yl and the like. The substituent of the optionally substituted nitrogen atom is exemplified by alkyl, aralkyl, haloalkyl and the like. As used herein, alkyl, aralkyl and haloalkyl are as defined for R and R¹.

Alkyl at R² is as defined for R and R¹.
Halogen, alkyl, alkoxy and aralkyl at R³ and R⁴ are as defined for R and R¹.
Acyl at R³ and R⁴ is alkanoyl having 2 to 6 carbon atoms (e.g., acetyl, propionyl, butyryl, valeryl, pivaloyl and the like), benzoyl or phenylalkanoyl wherein the alkanoyl moiety has 2 to 4 carbon atoms (e.g., phenylacetyl, phenylpropionyl, phenylbutyryl and the like).
Alkylamino at R³ and R⁴ is that wherein the alkyl moiety is linear or branched alkyl having 1 to 6 carbon atoms. Examples thereof include methylamino, ethylamino, propylamino, isopropylamino, butylamino, isobutylamino, sec-butylamino, tert-butylamino, pentylamino, hexylamino and the like.
Acylamino at R³ and R⁴ is that wherein acyl moiety is alkanoyl having 2 to 6 carbon atoms, benzyl or the alkanoyl moiety is phenylalkanoyl having 2 to 4 carbon atoms and the like, which is exemplified by acetylamino, propionylamino, butyrylamino, valerylamino, pivaloylamino, benzoylamino, phenylacetylamino, phenylpropionylamino, phenylbutyrylamino and the like.

Alkylthio at R³ and R⁴ is that wherein the alkyl moiety is linear or branched alkyl having 1 to 6 carbon atoms, which is exemplified by methylthio, ethylthio, propylthio, isopropylthio, butylthio, isobutylthio, sec-butylthio, tert-butylthio, pentylthio, hexylthio and the like.
Aralkyloxy at R³ and R⁴ is that wherein the alkyl moiety is alkyl having 1 to 4 carbon atoms, which is exemplified by benzyloxy, 1-phenylethyloxy, 2-phenylethyloxy, 3-phenylpropyloxy, 4-phenylbutyloxy and the like.
Aralkylthio at R³ and R⁴ is that wherein the alkyl moiety is alkyl having 1 to 4 carbon atoms, which is exemplified by benzylthio, 1-phenylethylthio, 2-phenylethylthio, 3-phenylpropylthio, 4-phenylbutylthio and the like.
Alkoxycarbonyl at R³ and R⁴ is that wherein the alkoxy moiety is linear or branched alkoxy having 1 to 6 carbon atoms, which is exemplified by methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl and the like.
Alkylcarbamoyl at R³ and R⁴ is carbamoyl mono- or disubstituted by alkyl having 1 to 4 carbon atoms, which is exemplified by methylcarbamoyl, dimethylcarbamoyl, ethylcarbamoyl, diethylcarbamoyl, propylcarbamoyl, dipropylcarbamoyl, butylcarbamoyl, dibutylcarbamoyl and the like.

Alkoxy at R⁵ is as defined for R and R¹.
Alkoxycarbonyloxy at R⁵ is that wherein the alkoxy moiety is linear or branched alkoxy having 1 to 6 carbon atoms, which is exemplified by methoxycarbonyloxy, ethoxycarbonyloxy, propoxycarbonyloxy, isopropoxycarbonyloxy, butoxycarbonyloxy, isobutoxycarbonyloxy, sec-butoxycarbonyloxy, tert-butoxycarbonyloxy, pentyloxycarbonyloxy, hexyloxycarbonyloxy and the like.
Alkanoyloxy at R⁵ is that wherein the alkanoyl moiety is alkanoyl having 2 to 6 carbon atoms, which is exemplified by acetyloxy, propionyloxy, butyryloxy, valeryloxy, pivaloyloxy and the like.
Aralkyloxycarbonyloxy at R⁵ is that wherein the aralkyl moiety is aralkyl having C₁-C₄ alkyl, which is exemplified by benzyloxycarbonyloxy, 1-phenylethyloxycarbonyloxy, 2-phenylethyloxycarbonyloxy, 3-phenylpropyloxycarbonyloxy, 4-phenylbutyloxycarbonyloxy and the like.

Alkyl at R⁶ is as defined for R and R¹; alkyl at R⁸ and R⁹ is as defined for R and R¹; and aralkyl at R⁸ and R⁹ is as defined for R and R¹.
Alkyl at R⁷ is as defined for R and R¹ and aralkyl at R⁷ is as defined for R and R¹.
The group formed by R⁶ and R⁷ in combination, which forms a heterocycle optionally further having, in the ring, oxygen atom, sulfur atom or optionally substituted nitrogen atom, is exemplified by imidazol-2-yl, thiazol-2-yl, oxazol-2-yl, imidazolin-2-yl, 3,4,5,6-tetrahydropyridin-2-yl, 3,4,5,6-tetrahydropyrimidin-2-yl, 1,3-oxazolin-2-yl, 1,3-thiazolin-2-yl or optionally substituted benzoimidazol-2-yl, benzothiazol-2-yl, benzoxazol-2-yl and the like having a substituent such as halogen, alkyl, alkoxy, haloalkyl, nitro, amino, phenyl, aralkyl and the like. As used herein, halogen, alkyl, alkoxy, haloalkyl and aralkyl are as defined for R and R¹.
The substituent of the above-mentioned optionally substituted nitrogen atom is exemplified by alkyl, aralkyl, haloalkyl and the like. As used herein, alkyl, aralkyl and haloalkyl are as defined for R and R¹.

Hydroxyalkyl at R¹⁰ and R¹¹ is linear or branched alkyl having 1 to 6 carbon atoms which is substituted by 1 to 3 hydroxy, which is exemplified by hydroxymethyl, 2-hydroxyethyl, 1-hydroxyethyl, 3-hydroxypropyl, 4-hydroxybutyl and the like. Alkyl at R¹⁰ and R¹¹ is as defined for R and R¹; haloalkyl and alkoxycarbonyl at R¹⁰ and R¹¹ are as defined for R and R¹; aralkyl at R¹⁰ and R¹¹ is as defined for R and R¹. Cycloalkyl formed by R¹⁰ and R¹¹ in combination is the same as cycloalkyl at R and R¹.

Alkyl at L is as defined for R and R¹.
Aminoalky at L is a linear or branched alkyl having 1 to 6 carbon atoms, which is substituted by amino, which is exemplified by aminomethyl, 2-aminoethyl, 1-aminoethyl, 3-aminopropyl, 4-aminobutyl, 5-aminopentyl, 6-aminohexyl and the like.
Mono- or dialkylaminoalkyl at L is mono- or disubstituted aminoalkyl with alkyl having 1 to 4 carbon atoms, which is exemplified by methylaminomethyl, dimethylaminomethyl, ethylaminomethyl, diethylaminomethyl, propylaminomethyl, dipropylaminomethyl, butylaminomethyl, dibutylaminomethyl, 2-dimethylaminoethyl, 2-diethylaminoethyl and the like.
Carbamoylalkyl at L is linear or branched alkyl having 1 to 6 carbon atoms substituted by carbamoyl, which is exemplified by carbamoylmethyl, 2-carbamoylethyl, 1-carbamoylethyl, 3-carbamoylpropyl, 4-carbamoylbutyl, 5-carbamoylpentyl, 6-carbamoylhexyl and the like.
Phthalimidoalkyl at L is linear or branched alkyl having 1 to 6 carbon atoms, which is substituted by phthalimide. Examples thereof include phthalimidomethyl, 2-phthalimidoethyl, 1-phthalimidoethyl, 3-phthalimidopropyl, 4-phthalimidobutyl, 5-phthalimidopentyl, 6-phthalimidohexyl and the like.

Alkyl at B is as defined for R and R¹.
Alkoxy at B is as defined for R and R¹.
Aralkyl at B is as defined for R and R¹.
Aralkyloxy at B is as defined for R³ and R⁴.
Aminoalkyl at B is as defined for L.
Hydroxyalkyl at B is as defined for R¹⁰ and R¹¹.
Alkanoyloxyalkyl at B is that wherein linear or branched alkyl having 1 to 6 carbon atoms is substituted by alkanoyloxy having alkanoyl moiety having 2 to 6 carbon atoms, which is exemplified by acetyloxymethyl, propionyloxymethyl, butyryloxymethyl, valeryloxymethyl, pivaloyloxymethyl, acetyloxyethyl, propionyloxyethyl, butyryloxyethyl, valeryloxyethyl, pivaloyloxyethyl and the like.
Alkoxycarbonylalkyl at B is that wherein linear or branched alkyl having 1 to 6 carbon atoms is substituted by alkoxycarbonyl having alkoxy moiety having 1 to 6 carbon atoms, which is exemplified by methoxycarbonylmethyl, ethoxycarbonylmethyl, propoxycarbonylmethyl, isopropoxycarbonylmethyl, butoxycarbonylmethyl, isobutoxycarbonylmethyl, sec-butoxycarbonylmethyl, tert-butoxycarbonylmethyl, pentyloxycarbonylmethyl, hexyloxycarbonylmethyl, methoxycarbonylethyl, ethoxycarbonylethyl, propoxycarbonylethyl, isopropoxycarbonylethyl, butoxycarbonylethyl, isobutoxycarbonylethyl, sec-butoxycarbonylethyl, tert-butoxycarbonylethyl, pentyloxycarbonylethyl, hexyloxycarbonylethyl and the like.

Halogen at Q¹, Q² and Q³ is as defined for R and R¹.
Aralkyloxy at Q¹ and Q² is as defined for R³ and R⁴.
Alkoxy at Q³ is as defined for R and R¹.
Alkylene at W, X and Y is linear or branched alkylene having 1 to 6 carbon atoms, which is exemplified by methylene, ethylene, trimethylene, propylene, tetramethylene, pentamethylene, hexamethylene and the like.
Alkenylene at Y is linear or branched alkenylene having 2 to 6 carbon atoms, which is exemplified by vinylene, propenylene, butenylene, pentenylene and the like.

Alkyl at Rb is as defined for R and R¹.
Aralkyl at Rb is as defined for R and R¹.
Aminoalkyl at Rb is as defined for L.
Mono- or dialkylaminoalkyl at Rb is as defined for L.

The nitrogen-containing heterocycle at Rc, when it is a monocyclic ring, is exemplified by pyridine, pyrimidine, pyridazine, triazine, pyrazole, triazole and the like, and when it is a condensed ring, it is exemplified by pyrrolopyridine (e.g., 1H-pyrrolo[2,3-b]pyridine, 1H-pyrrolo[3,2-b]pyridine, 1H-pyrrolo[3,4-b]pyridine and the like), pyrazolopyridine (e.g., 1H-pyrazolo[3,4-b]pyridine, 1H-pyrazolo[4,3-b]pyridine and the like), imidazopyridine (e.g., 1H-imidazo[4,5-b]pyridine and the like), pyrrolopyrimidine (e.g., 1H-pyrrolo[2,3-d]pyrimidine, 1H-pyrrolo[3,2-d]pyrimidine, 1H-pyrrolo[3,4-d]pyrimidine and the like), pyrazolopyrimidine (e.g., 1H-pyrazolo[3,4-d]pyrimidine, pyrazolo[1,5-a]pyrimidine, 1H-pyrazolo[4,3-d]pyrimidine and the like), imidazopyrimidine (e.g., imidazo[1,2-a]pyrimidine, 1H-imidazo[4,5-d]pyrimidine and the like), pyrrolotriazine (e.g., pyrrolo[1,2-a]-1,3,5-triazine, pyrrolo[2,1-f]-1,2,4-triazine), pyrazolotriazine (e.g., pyrazolo[1,5-a]-1,3,5-triazine and the like), triazolopyridine (e.g., 1H-1,2,3-triazolo[4,5-b]pyridine and the like), triazolopyrimidine (e.g., 1,2,4-triazolo[1,5-a]pyrimidine, 1,2,4-triazolo[4,3-a]pyrimidine, 1H-1,2,3-triazolo[4,5-d]pyrimidine and the like), cinnoline, quinazoline, quinoline, pyridopyridazine (e.g., pyrido[2,3-c]pyridazine and the like), pyridopyrazine (e.g., pyrido[2,3-b]pyrazine and the like), pyridopyrimidine (e.g., pyrido[2,3-d]pyrimidine, pyrido[3,2-d]pyrimidine and the like), pyrimidopyrimidine (e.g., pyrimido[4,5-d]pyrimidine, pyrimido[5,4-d]pyrimidine and the like), pyrazinopyrimidine (e.g., pyrazino[2,3-d]pyrimidine and the like), naphthyridine (e.g., 1,8-naphthyridine and the like), tetrazolopyrimidine (e.g., tetrazolo[1,5-a]pyrimidine and the like), thienopyridine (e.g., thieno[2,3-b]pyridine and the like), thienopyrimidine (e.g., thieno[2,3-d]pyrimidine and the like), thiazolopyridine (e.g., thiazolo[4,5-b]pyridine, thiazolo[5,4-b]pyridine and the like), thiazolopyrimidine (e.g., thiazolo[4,5-d]pyrimidine, thiazolo[5,4-d]pyrimidine and the like), oxazolopyridine (e.g., oxazolo[4,5-b]pyridine, oxazolo[5,4-b]pyridine and the like), oxazolopyrimidine (e.g., oxazolo[4,5-d]pyrimidine, oxazolo[5,4-d]pyrimidine and the like), furopyridine (e.g., furo[2,3-b]pyridine, furo[3,2-b]pyridine and the like), furopyrimidine (e.g., furo[2,3-d]pyrimidine, furo[3,2-d]pyrimidine and the like), 2,3-dihydropyrrolopyridine (e.g., 2,3-dihydro-1H-pyrrolo[2,3-b]pyridine, 2,3-dihydro-1H-pyrrolo[3,2-b]pyridine and the like), 2,3-dihydropyrrolopyrimidine (e.g., 2,3-dihydro-1H-pyrrolo[2,3-d]pyrimidine, 2,3-dihydro-1H-pyrrolo[3,2-d]pyrimidine and the like), 5,6,7,8-tetrahydropyrido[2,3-d]pyrimidine, 5,6,7,8-tetrahydro-1,8-naphthyridine, 5,6,7,8-tetrahydroquinoline and the like. When these rings form a hydrogenated aromatic ring, the carbon atom in the ring may be carbonyl and includes, for example, 2,3-dihydro-2-oxopyrrolopyridine, 2,3-dihydro-2,3-dioxopyrrolopyridine, 7,8-dihydro-7-oxo-1,8-naphthyridine, 5,6,7,8-tetrahydro-7-oxo-1,8-naphthyridine and the like. Preferably is pyridine, pyrrolopyridine.

These rings may be substituted by a substituent such as halogen, alkyl, alkoxy, aralkyl, haloalkyl, nitro, amino, alkylamino, cyano, formyl, acyl, aminoalkyl, mono- or dialkylaminoalkyl, azide, carboxy, alkoxycarbonyl, carbamoyl, alkylcarbamoyl, alkoxyalkyl (e.g., methoxymethyl, methoxyethyl, methoxypropyl, ethoxymethyl, ethoxyethyl, ethoxypropyl and the like), optionally substituted hydrazino and the like.
As used herein, the substituent of the optionally substituted hydrazino includes alkyl, aralkyl, nitro, cyano and the like, wherein alkyl and aralkyl are as defined for R and R¹ and exemplified by methylhydrazino, ethylhydrazino, benzylhydrazino and the like.

The compound of the formula (I) is exemplified by the following compounds and salts thereof.
(1) 4-(2-pyridylcarbamoyl)piperidine
(2) 1-benzyloxycarbonyl-4-(4-pyridylcarbamoyl)piperidine
(3) 1-benzoyl-4-(4-pyridylcarbamoyl)piperidine
(4) 1-propyl-4-(4-pyridylcarbamoyl)piperidine
(5) [3-(2-(2-thienylmethyl)phenoxy)-2-hydroxypropyl]-4-(4-pyridylcarbamoyl)piperidine
(6) 4-(4-pyridylcarbamoyl)piperidine
(7) 1-benzyl-4-(4-pyridylcarbamoyl)-1,2,5,6-tetrahydropyridine
(8) 3-(4-pyridylcarbamoyl)piperidine
(9) 1-benzyl-3-(4-pyridylcarbamoyl)piperidine
(10) 1-(2-(4-benzyloxyphenoxy)ethyl)-4-(N-(2-pyridyl)-N-benzylcarbamoyl)piperidine
(11) 1-formyl-4-(4-pyridylcarbamoyl)piperidine
(12) 4-(3-pyridylcarbamoyl)piperidine
(13) 1-isopropyl-4-(4-pyridylcarbamoyl)piperidine
(14) 1-methyl-4-(4-pyridylcarbamoyl)piperidine
(15) 1-hexyl-4-(4-pyridylcarbamoyl)piperidine
(16) 1-benzyl-4-(4-pyridylcarbamoyl)piperidine
(17) 1-(2-phenylethyl)-4-(4-pyridylcarbamoyl)piperidine
(18) 1-(2-(4-methoxyphenyl)ethyl)-4-(4-pyridylcarbamoyl)-piperidine
(19) 1-(2-(4-methoxyphenyl)ethyl)-4-(2-pyridylcarbamoyl)-piperidine
(20) 1-(2-(4-chlorophenyl)ethyl)-4-(4-pyridylcarbamoyl)-piperidine

(21) 1-diphenylmethyl-4-(2-pyridylcarbamoyl)piperidine
(22) 1-[2-(4-(5-methyl-3-oxo-2,3,4,5-tetrahydropyridazin-6-yl)phenyl)ethyl]-4-(2-pyridylcarbamoyl)piperidine
(23) 1-(4-(4,5-dihydro-2-furyl)phenyl)-4-(4-pyridylcarbamoyl)piperidine
(24) 1-(2-nitrophenyl)-4-(4-pyridylcarbamoyl)piperidine
(25) 1-(2-aminophenyl)-4-(4-pyridylcarbamoyl)piperidine
(26) 1-nicotinoyl-4-(4-pyridylcarbamoyl)piperidine
(27) 1-isonicotinoyl-4-(4-pyridylcarbamoyl)piperidine
(28) 1-(3,4,5-trimethoxybenzoyl)-4-(4-pyridylcarbamoyl)-piperidine
(29) 1-acetyl-4-(4-pyridylcarbamoyl)piperidine
(30) 1-(3-(4-fluorobenzoyl)propyl)-4-(4-pyridylcarbamoyl)-piperidine
(31) 1-(3-(4-fluorobenzoyl)propyl)-4-(2-pyridylcarbamoyl)-piperidine
(32) 1-(1-(4-hydroxybenzoyl)ethyl)-4-(2-pyridylcarbamoyl)-piperidine
(33) 1-(1-(4-benzyloxybenzoyl)ethyl)-4-(2-pyridylcarbamoyl)-piperidine
(34) 1-(2-(4-hydroxyphenoxy)ethyl)-4-(2-pyridylcarbamoyl)-piperidine
(35) 1-(4-(4-fluorophenyl)-4-hydroxybutyl)-4-(4-pyridylcarbamoyl)piperidine
(36) 1-(1-methyl-2-(4-hydroxyphenyl)-2-hydroxyethyl)-4-(2-pyridylcarbamoyl)piperidine
(37) 1-cinnamyl-4-(2-pyridylcarbamoyl)piperidine
(38) 1-(2-hydroxy-3-phenoxypropyl)-4-(4-pyridylcarbamoyl)-piperidine
(39) 1-(2-hydroxy-3-phenoxypropyl)-4-(3-pyridylcarbamoyl)-piperidine
(40) 1-(2-hydroxy-3-phenoxypropyl)-4-(2-pyridylcarbamoyl)-piperidine

(41) 1-(2-phenylethyl)-4-[N-(2-pyridyl)-N-(2-(N,N-dimethylamino)ethyl)carbamoyl]piperidine
(42) 1-benzyloxycarbonyl-4-(2-pyridylcarbamoyl)piperidine
(43) 1-(3-chlorophenyl)carbamoyl-4-(4-pyridylcarbamoyl)-piperidine
(44) 1-[N-(2-pyridyl)-N-(2-(N,N-dimethylamino)ethyl)-carbamoyl]piperidine
(45) 1-methyl-4-(4-pyridylcarbamoyl)-1,2,5,6-tetrahydropyridine
(46) 1-nicotinoyl-3-(4-pyridylcarbamoyl)piperidine
(47) 1-[2-(4-fluorobenzoyl)ethyl]-4-(4-pyridylcarbamoyl)-piperidine
(48) 1-(6-chloro-2-methylimidazo[1,2-a]pyridine-3-carbonyl)-4-(4-pyridylcarbamoyl)piperidine
(49) 1-(4-nitrobenzyl)-4-(4-pyridylcarbamoyl)piperidine
(50) 1-hexyl-4-(4-pyridylcarbamoyl)piperidine
(51) 1-benzyloxycarbonyl-4-(2-chloro-4-pyridylcarbamoyl)-piperidine
(52) 4-(2-chloro-4-pyridylcarbamoyl)piperidine
(53) 1-(2-chloronicotinoyl)-4-(4-pyridylcarbamoyl)piperidine
(54) 3-(2-chloro-4-pyridylcarbamoyl)piperidine
(55) 1-(4-phthalimidobutyl)-4-(4-pyridylcarbamoyl)piperidine
(56) 1-(3,5-di-tert-butyl-4-hydroxycinnamoyl)-4-(4-pyridylcarbamoyl)piperidine
(57) 1-carbamoylmethyl-4-(4-pyridylcarbamoyl)piperidine
(58) 1-benzyloxycarbonyl-4-(5-nitro-2-pyridylcarbamoyl)-piperidine
(59) 4-(5-nitro-2-pyridylcarbamoyl)piperidine
(60) trans-4-benzyloxycarboxamidomethyl-1-(4-pyridylcarbamoyl)cyclohexane

(61) trans-4-aminomethyl-1-(4-pyridylcarbamoyl)cyclohexane
(62) trans-4-formamidomethyl-1-(4-pyridylcarbamoyl)-cyclohexane
(63) trans-4-dimethylaminomethyl-1-(4-pyridylcarbamoyl)-cyclohexane
(64) N-benzylidene-trans-(4-pyridylcarbamoyl)-cyclohexylmethylamine
(65) trans-4-benzylaminomethyl-1-(4-pyridylcarbamoyl)-cyclohexane
(66) trans-4-isopropylaminomethyl-1-(4-pyridylcarbamoyl)-cyclohexane
(67) trans-4-nicotinoylaminomethyl-1-(4-pyridylcarbamoyl)-cyclohexane
(68) trans-4-cyclohexylaminomethyl-1-(4-pyridylcarbamoyl)-cyclohexane
(69) trans-4-benzyloxycarboxamide-1-(4-pyridylcarbamoyl)-cyclohexane
(70) trans-4-amino-1-(4-pyridylcarbamoyl)cyclohexane
(71) trans-4-(1-aminoethyl)-1-(4-pyridylcarbamoyl)cyclohexane
(72) trans-4-aminomethyl-cis-2-methyl-1-(4-pyridylcarbamoyl)-cyclohexane
(73) (+)-trans-4-(1-benzyloxycarboxamidopropyl)-1-cyclohexanecarboxylic acid
(74) (+)-trans-4-(1-benzyloxycarboxamidopropyl)-1-(4-pyridylcarbamoyl)cyclohexane
(75) (-)-trans-4-(1-benzyloxycarboxamidopropyl)-1-(4-pyridylcarbamoyl)cyclohexane
(76) (+)-trans-4-(1-aminopropyl)-1-(4-pyridylcarbamoyl)-cyclohexane
(77) (-)-trans-4-(1-aminopropyl)-1-(4-pyridylcarbamoyl)-cyclohexane
(78) (-)-trans-4-(1-benzyloxycarboxamidoethyl)-1-(4-pyridylcarbamoyl)cyclohexane
(79) (+)-trans-4-(1-benzyloxycarboxamidoethyl)-1-(4-pyridylcarbamoyl)cyclohexane
(80) (+)-trans-4-(1-aminoethyl)-1-(4-pyridylcarbamoyl)-cyclohexane

(81) (-)-trans-4-(1-aminoethyl)-1-(4-pyridylcarbamoyl)-cyclohexane
(82) trans-4-(4-chlorobenzoyl)aminomethyl-1-(4-pyridylcarbamoyl)cyclohexane
(83) trans-4-aminomethyl-1-(2-pyridylcarbamoyl)cyclohexane
(84) trans-4-benzyloxycarboxamidomethyl-1-(2-pyridylcarbamoyl)cyclohexane
(85) trans-4-methylaminomethyl-1-(4-pyridylcarbamoyl)-cyclohexane
(86) trans-4-(N-benzyl-N-methylamino)methyl-1-(4-pyridylcarbamoyl)cyclohexane
(87) trans-4-aminomethyl-1-(3-pyridylcarbamoyl)cyclohexane
(88) trans-4-aminomethyl-1-[(3-hydroxy-2-pyridyl)carbamoyl]-cyclohexane
(89) trans-4-benzyloxycarboxamidomethyl-1-(3-pyridylcarbamoyl)cyclohexane
(90) trans-4-benzyloxycarboxamidomethyl-1-[(3-benzyloxy-2-pyridyl)carbamoyl]cyclohexane
(91) trans-4-phthalimidomethyl-1-(4-pyridylcarbamoyl)-cyclohexane
(92) trans-4-benzyloxycarboxamidomethyl-1-(3-methyl-4-pyridylcarbamoyl)cyclohexane
(93) trans-4-aminomethyl-1-(3-methyl-4-pyridylcarbamoyl)-cyclohexane
(94) 4-(trans-4-benzyloxycarboxamidomethylcyclohexylcarbonyl)amino-2,6-dimethylpyridine-N-oxide
(95) 4-(trans-4-aminomethylcyclohexylcarbonyl)amino-2,6-dimethylpyridine-N-oxide
(96) trans-4-aminomethyl-1-(2-methyl-4-pyridylcarbamoyl)-cyclohexane
(97) trans-4-(1-benzyloxycarboxamidoethyl)-1-(4-pyridylcarbamoyl)cyclohexane
(98) trans-4-(1-amino-1-methylethyl)-1-(4-pyridylcarbamoyl)-cyclohexane
(99) trans-4-(2-aminoethyl)-1-(4-pyridylcarbamoyl)cyclohexane
(100) trans-4-(2-amino-1-methylethyl)-1-(4-pyridylcarbamoyl)cyclohexane

(101) trans-4-(1-aminopropyl)-1-(4-pyridylcarbamoyl)-cyclohexane
(102) trans-4-aminomethyl-trans-1-methyl-1-(4-pyridylcarbamoyl)cyclohexane
(103) trans-4-benzylaminomethyl-cis-2-methyl-1-(4-pyridylcarbamoyl)cyclohexane
(104) trans-4-(1-benzyloxycarboxamide-1-methylethyl)-1-(4-pyridylcarbamoyl)cyclohexane
(105) trans-4-benzyloxycarboxamidomethyl-1-(N-methyl-4-pyridylcarbamoyl)cyclohexane
(106) trans-4-(1-acetamide-1-methylethyl)-1-(4-pyridylcarbamoyl)cyclohexane
(107) trans-N-(6-amino-4-pyrimidyl)-4-aminomethylcyclohexanecarboxamide
(108) trans-N-(1H-pyrrolo[2,3-b]pyridin-4-yl)-4-aminomethylcyclohexanecarboxamide
(109) (+)-trans-N-(1H-pyrrolo[2,3-b]pyridin-4-yl)-4-(1-aminoethyl)cyclohexanecarboxamide
(110) trans-N-(1H-pyrrolo[2,3-b]pyridin-4-yl)-4-(1-amino-1-methylethyl)cyclohexanecarboxamide
(111) trans-N-(1H-pyrazolo[3,4-b]pyridin-4-yl)-4-aminomethylcyclohexanecarboxamide
(112) (+)-trans-N-(1H-pyrazolo[3,4-b]pyridin-4-yl)-4-(1-aminoethyl)cyclohexanecarboxamide
(113) trans-N-(1H-pyrazolo[3,4-b]pyridin-4-yl)-4-(1-amino-1-methylethyl)cyclohexanecarboxamide
(114) (+)-trans-N-(2-amino-4-pyridyl)-4-(1-aminoethyl)-cyclohexanecarboxamide
(115) trans-N-(1H-pyrazolo[3,4-d]pyrimidin-4-yl)-4-aminomethylcyclohexanecarboxamide
(116) (+)-trans-N-(1H-pyrazolo[3,4-d]pyrimidin-4-yl)-4-(1-aminoethyl)cyclohexanecarboxamide
(117) trans-N-(1H-pyrazolo[3,4-d]pyrimidin-4-yl)-4-(1-amino-1-methylethyl)cyclohexanecarboxamide
(118) trans-N-(4-pyrimidinyl)-4-aminomethylcyclohexanecarboxamide
(119) trans-N-(3-amino-4-pyridyl)-4-aminomethylcyclohexanecarboxamide
(120) trans-N-(7H-imidazo[4,5-d]pyrimidin-6-yl)-4-aminomethylcyclohexanecarboxamide

(121) trans-N-(3H-1,2,3-triazolo[4,5-dlpyrimidin-7-yl)-4-aminomethylcyclohexanecarboxamide
(122) trans-N-(1-benzyl-1H-pyrazolo[3,4-b]pyridin-4-yl)-4-aminomethylcyclohexanecarboxamide
(123) trans-N-(1H-5-pyrazolyl)-4-aminomethylcyclohexanecarboxamide
(124) trans-N-(1H-pyrazolo[3,4-b]pyridin-4-yl)-4-aminomethylcyclohexanecarboxamide
(125) trans-N-(4-pyridazinyl)-4-aminomethylcyclohexanecarboxamide
(126) trans-N-(7H-pyrrolo[2,3-d]pyrimidin-4-yl)-4-aminomethylcyclohexanecarboxamide
(127) trans-N-(2-amino-4-pyridyl)-4-aminomethylcyclohexanecarboxamide
(128) trans-N-(thieno[2,3-d]pyrimidin-4-yl)-4-aminomethylcyclohexanecarboxamide
(129) trans-N-(5-methyl-1,2,4-triazolo[1,5-a]pyrimidin-7-yl)-4-aminomethylcyclohexanecarboxamide
(130) trans-N-(3-cyano-5-methylpyrazolo[1,5-a]pyrimidin-7-yl)-4-aminomethylcyclohexanecarboxamide
(131) trans-N-(1H-pyrazolo[3,4-b]pyridin-4-yl)-4-(1-amino-1-methylethyl)cyclohexanecarboxamide
(132) trans-N-(2-(1-pyrrolidinyl)-4-pyridyl)-4-aminomethylcyclohexanecarboxamide
(133) trans-N-(2,6-diamino-4-pyrimidyl)-4-aminomethylcyclohexanecarboxamide
(134) (+)-trans-N-(7-methyl-1,8-naphthyridin-4-yl)-4-(1-aminoethyl)cyclohexanecarboxamide
(135) trans-N-(1-benzyloxymethylpyrrolo[2,3-b]pyridin-4-yl)-4-aminomethylcyclohexanecarboxamide
(136) (+)-trans-N-(1-methylpyrrolo[2,3-b]pyridin-4-yl)-4-(1-aminoethyl)cyclohexanecarboxamide
(137) trans-N-benzyl-N-(2-benzylamino-4-pyridyl)-4-(1-amino-1-methylethyl)cyclohexanecarboxamide
(138) trans-N-(2-azide-4-pyridyl)-4-aminomethylcyclohexanecarboxamide
(139) trans-N-(2,3-dihydro-1H-pyrrolo[2,3-b]pyridin-4-yl)-4-aminomethylcyclohexanecarboxamide
(140) trans-N-(2,3-dihydro-1H-pyrrolo[2,3-b]pyridin-4-yl)-4-(1-amino-1-methylethyl)cyclohexanecarboxamide

(141-1) trans-N-(2-carboxy-4-pyridyl)-4-aminomethylcyclohexanecarboxamide
(141-2) (R)-(+)-trans-N-(3-bromo-1H-pyrrolo[2,3-b]pyridin-4-yl)-4-(1-aminoethyl)cyclohexanecarboxamide
(142) trans-N-(1H-pyrrolo[2,3-b]pyridin-4-yl)-4-guanidinomethylcyclohexanecarboxamide
(143) trans-N-(1H-pyrazolo[3,4-b]pyridin-4-yl)-4-guanidinomethylcyclohexanecarboxamide
(144) trans-N-(4-pyridyl)-4-guanidinomethylcyclohexanecarboxamide
(145) trans-N-(1-methylpyrrolo[2,3-b]pyridin-4-yl)-4-(guanidinomethyl)cyclohexanecarboxamide
(146) trans-N-(1H-pyrrolo[2,3-b]pyridin-4-yl)-4-(2-imidazolin-2-yl)aminomethylcyclohexanecarboxamide
(147) trans-N-(1-benzyloxymethylpyrrolo[2,3-b]pyridin-4-yl)-4-guanidinomethylcyclohexanecarboxamide
(148) trans-N-(2-amino-4-pyridyl)-4-guanidinomethylcyclohexanecarboxamide
(149) trans-N-(1-benzyloxymethyl-1H-pyrrolo[2,3-b]pyridin-4-yl)-4-(2-imidazolin-2-yl)aminomethylcyclohexanecarboxamide
(150) trans-N-(1H-pyrrolo[2,3-b]pyridin-4-yl)-4-(3-benzylguanidinomethyl)cyclohexanecarboxamide
(151) trans-N-(1H-pyrrolo[2,3-b]pyridin-4-yl)-4-(3-phenylguanidinomethyl)cyclohexanecarboxamide
(152) trans-N-(1H-pyrrolo[2,3-b]pyridin-4-yl)-4-(3-propylguanidinomethyl)cyclohexanecarboxamide
(153) trans-N-(1H-pyrrolo[2,3-b]pyridin-4-yl)-4-(3-octylguanidinomethyl)cyclohexanecarboxamide
(154) trans-N-(1-benzyloxymethylpyrrolo[2,3-b]pyridin-4-yl)-4-(2-benzyl-3-ethylguanidinomethyl)cyclohexanecarboxamide
(155) trans-N-(1H-pyrrolo[2,3-b]pyridin-4-yl)-4-(imidazol-2-yl)aminomethylcyclohexanecarboxamide
(156) trans-N-(1H-pyrrolo[2,3-b]pyridin-4-yl)-4-(thiazol-2-yl)aminomethylcyclohexanecarboxamide
(157) (R)-(+)-N-(4-pyridyl)-4-(1-aminoethyl)benzamide
(158) N-(4-pyridyl)-4-(1-amino-1-methylethyl)benzamide
(159) N-(4-pyridyl)-4-aminomethyl-2-benzyloxybenzamide
(160) N-(4-pyridyl)-4-aminomethyl-2-ethoxybenzamide

(161) (R)-(-)-N-(4-pyridyl)-4-(1-aminoethyl)-3-nitrobenzamide
(162) (R)-(-)-N-(4-pyridyl)-3-amino-4-(1-aminoethyl)benzamide
(163) (R)-(+)-N-(4-pyridyl)-4-(1-aminoethyl)-3-chlorobenzamide
(164) N-(4-pyridyl)-3-aminomethylbenzamide
(165) (R)-(+)-N-(1H-pyrrolo[2,3-b]pyridin-4-yl)-4-(1-aminoethyl)benzamide
(166) (R)-(+)-N-(1H-pyrazolo[3,4-b]pyridin-4-yl)-4-(1-aminoethyl)benzamide
(167) N-(1H-pyrazolo[3,4-b]pyridin-4-yl)-4-guanidinomethylbenzamide
(168) N-(4-pyridyl)-4-guanidinomethylbenzamide
(169) (R)-(+)-N-(4-pyridyl)-4-(1-aminoethyl)-3-fluorobenzamide
(170) N-(4-pyridyl)-4-aminomethylbenzamide
(171) N-(4-pyridyl)-4-aminomethyl-2-hydroxybenzamide
(172) N-(4-pyridyl)-4-(2-aminoethyl)benzamide
(173) N-(4-pyridyl)-4-aminomethyl-3-nitrobenzamide
(174) N-(4-pyridyl)-3-amino-4-aminomethylbenzamide
(175) (S)-(-)-N-(4-pyridyl)-4-(1-aminoethyl)benzamide
(176) (S)-(-)-N-(4-pyridyl)-2-(1-aminoethyl)benzamide
(177) (R)-(+)-N-(4-pyridyl)-4-(1-aminoethyl)-2-chlorobenzamide
(178) (R)-(+)-N-(1H-pyrrolo[2,3-b]pyridin-4-yl)-4-(1-(3-propylguanidino)ethyl)benzamide
(179) (R)-(-)-N-(1H-pyrrolo[2,3-b]pyridin-4-yl)-4-(1-aminoethyl)-3-azidebenzamide
(180) (R)-(+)-N-(4-pyridyl)-4-(1-aminoethyl)-2-nitrobenzamide

(181) (R)-(-)-N-(4-pyridyl)-4-(1-aminoethyl)-3-ethoxybenzamide
(182) (R)-(+)-N-(3-iodo-1H-pyrrolo[2,3-b]pyridin-4-yl)-4-(1-aminoethyl)benzamide
(183) (R)-(+)-N-(3-iodo-1H-pyrrolo[2,3-b]pyridin-4-yl)-4-(1-aminoethyl)-3-azidebenzamide
(184) (R)-(-)-N-(4-pyridyl)-4-(1-aminoethyl)-3-hydroxybenzamide
(185) N-(1H-pyrazolo[3,4-b]pyridin-4-yl)-4-guanidinomethyl-3-nitrobenzamide
(186) (R)-N-(1H-pyrazolo[3,4-b]pyridin-4-yl)-4-(1-guanidinoethyl)-3-nitrobenzamide
(187) (R)-N-(1H-pyrazolo[3,4-b]pyridin-4-yl)-4-(1-aminoethyl)-2-nitrobenzamide
(188) N-(1H-pyrazolo[3,4-b]pyridin-4-yl)-4-guanidinobenzamide (189) (R)-N-(1H-pyrazolo[3,4-b]pyridin-4-yl)-4-(1-aminoethyl)-3-nitrobenzamide
(190) (R)-N-(1H-pyrazolo[3,4-b]pyridin-4-yl)-4-(1-guanidinoethyl)benzamide
(191) N-(1H-pyrazolo[3,4-b]pyridin-4-yl)-4-(1-amino-2-hydroxyethyl)benzamide
(192) N-(1H-pyrazolo[3,4-b]pyridin-4-yl)-4-aminomethyl-3-nitrobenzamide
(193) N-(1H-pyrrolo[2,3-b]pyridin-4-yl)-4-piperidinecarboxamide
(194) N-(1H-pyrazolo[3,4-b]pyridin-4-yl)-4-piperidinecarboxamide
(195) N-(1H-pyrazolo[3,4-b]pyridin-4-yl)-1-aminoacetyl-4-piperidinecarboxamide
(196) N-(1-methoxymethyl-1H-pyrazolo[3,4-b]pyridin-4-yl)-4-piperidinecarboxamide
(197) N-(2,3-dihydro-1H-pyrrolo[2,3-b]pyridin-4-yl)-4-piperidinecarboxamide
(198) N-(1H-pyrrolo[2,3-b]pyridin-4-yl)-1-(2-phenylethyl)-4-piperidinecarboxamide
(199) N-(1H-pyrrolo[2,3-b]pyridin-4-yl)-1-amidino-4-piperidinecarboxamide
(200) N-(1H-pyrrolo[2,3-b]pyridin-4-yl)-1-(3-phenylpropyl)-4-piperidinecarboxamide

(201) N-(1H-pyrrolo[2,3-b]pyridin-4-yl)-1-benzyl-4-piperidinecarboxamide
(202) N-(1H-pyrazolo[3,4-b]pyridin-4-yl)-1-(2-phenylethyl)-4-piperidinecarboxamide
(203) N-(1H-pyrazolo[3,4-b]pyridin-4-yl)-1-(3-phenylpropyl)-4-piperidinecarboxamide
Preferred are compounds (80), (109), (110), (112), (115), (142), (143), (144), (145), (153), (157), (163), (165), (166) and (179).

The compound may be a pharmaceutically acceptable acid addition salt, wherein the acid is exemplified by inorganic acid such as hydrochloric acid, hydrobromic acid, sulfuric acid and the like, and organic acid such as methanesulfonic acid, fumaric acid, maleic acid, mandelic acid, citric acid, tartaric acid, salicylic acid and the like. A compound having a carboxyl group can be converted to a salt with a metal such as sodium, potassium, calcium, magnesium, aluminum and the like, a salt with an amino acid such as lysine and the like. Further, monohydrate, dihydrate, 1/2 hydrate, 1/3 hydrate, 1/4 hydrate, 2/3 hydrate, 3/2 hydrate, 6/5 hydrate and the like are encompassed in the present invention. Preferably, the compound is (R)-(+)-N(1H-pyrrolo[2,3-b]pyridin-4-yl)-4-(1-aminoethyl)benzamide monohydrochloride.

The compound can be synthesized by a method described in, for example, JP-A-62-89679, JP-A-3-218356, JP-A-5-194401, JP-A-6-41080, WO95/28387, WO98/06433 and the like.

When the amide compound represented by the formula (I) has an optical isomer, its racemate or cis-trans isomers, all of them can be used in the present invention. These isomers can be isolated by a conventional method or can be produced using starting materials of the isomers.

The present compound has intraocular pressure lowering action, optic disc blood flow improving action and aqueous outflow promoting action in mammals inclusive of human, cow, horse, dog, mouse, rat and the like. Therefore, they can be used as an agent for the prophylaxis and treatment of various types of glaucoma, such as primary open angle glaucoma, normal pressure glaucoma, hypersecretion glaucoma, ocular hypertension, acute angle closure glaucoma, chronic angle closer glaucoma, plateau iris syndrome, combined-mechanism glaucoma, steroid glaucoma, capsular glaucoma, pigmentary glaucoma, secondary glaucoma associated with amyloidosis, neovascular glaucoma, malignant glaucoma and the like.
Since the present compound suppresses contraction of ciliary muscle in mammals such as human, bovine, horse, dog, mouse, rat and the like, the aqueous liquid preparation of the present invention is used as an agent for the prophylaxis or treatment of asthenopia or pseudomyopia caused by sustained abnormal tension of ciliary muscle and the like.
Since the present compound has axon of the retinal ganglion cell extending action and optic nerve cell regenerating action in mammal including human, bovine, horse, dog, mouse, rat and the like, a compound is considered to improve visual functional disorders caused by damage, degeneration and the like of the retinal nerve and the optic nerve. Accordingly, the aqueous liquid preparation of the present invention is considered to be effective for the improvement of visual function in a visual disorder caused by damage due to retinal inflammation and the like (retinal neuropathy, retinal vascular occlusion, periphlebitis retinae, Eales' disease, ischemic ophthalmopathy, retinal arteriolar microaneurysm, retinopathy caused by hypertension, renal disease and blood disease, diabetic retinopathy, retinal dystrophy, macular dystrophy, chorioretinopathy, macular degeneration, macular edema, retinal pigment epithelium detachment, degenerative retinoschisis, retinoblastoma, retinal pigment epithelioma etc.) and the like; improvement of visual function in a visual disorder caused by degeneration, damage of the optic nerve (optic neuritis, capillary angioma of optic disc, ischemic optic neuropathy, defects of retinal nerve fibers layer, retinal optic atrophy, neurotmesis of optic nerve, traumatic optic neuropathy, choked disc, coloboma of optic disc, optic nerve hypoplasia, toxic optic atrophy etc.); improvement of visual function in a visual disorder due to optic atrophy, degeneration and the like caused by elevated intraocular pressure (glaucoma etc.) and the like; and further, proliferation and functional maintenance of visual cells including retinal ganglion cells in retinal transplantation as well as regeneration of optic nerve in optic nerve transplantation.
Since the present compound promotes formation of corneal nerve protrusion in mammals such as human, bovine, horse, dog, mouse, rat and the like, the aqueous liquid preparation of the present invention is useful for the improvement of corneal sensitivity dysfunction caused by damage to corneal nerve or dry eye caused by corneal sensitivity dysfunction.
Specifically, it is used as an agent for the prophylaxis or treatment of corneal sensitivity dysfunction after cataract surgery, laser photorefractive keratectomy (PRK), laser keratomileusis (LASIK), LASEK or corneal transplantation, corneal sensitivity dysfunction caused by cornea neurodegeneration such as neuroparalytic keratopathy, corneal ulcer, diabetic keratopathy and the like, dry eye symptom and the like.

The aqueous liquid preparation of the present invention characteristically contains an ion-pairing reagent to promote intraocular penetration of the active ingredient.
In the present invention, the ion-pairing reagent refers to one that forms an ion-pair with the present compound in a liquid phase. The ion-pairing reagent is appropriately selected according to the pH of the aqueous liquid preparation of the present invention such that an ion-pair can be formed with the present compound (the active ingredient) in a liquid phase. For example, when pH is around 7, sorbic acid, octanoic acid, N-lauroyl-L-glutamic acid, dehydroacetic acid or saccharin or a pharmacologically acceptable salt thereof can be used, with preference given to sorbic acid, octanoic acid or N-lauroyl-L-glutamic acid or a pharmacologically acceptable salt thereof. When pH is around 5, acetyltryptophan, saccharin, sorbic acid, octanoic acid or dehydroacetic acid or a pharmacologically acceptable salt thereof can be used, with preference given to saccharin or a pharmacologically acceptable salt thereof. Examples of the aforementioned salt include alkali metal salts such as sodium salt, potassium salt and the like.

When the aqueous liquid preparation of the present invention is used as an eye drop, the pH of the liquid preparation is preferably adjusted to around 7. Thus, the ion-pairing reagent is preferably selected from sorbic acid, octanoic acid, N-lauroyl-L-glutamic acid and a pharmacologically acceptable salt thereof.

The mixing ratio of the present compound and the aforementioned ion-pairing reagent in a molar ratio is generally the present compound:ion-pairing reagent=1:0.01 - 1:100, preferably 1:0.1 - 1:10. An ion-pair is effectively formed in the aqueous liquid preparation of the present invention depending on the selection of the ion-pairing reagent and the above-mentioned mixing ratio, and the intraocular penetration of the present compound can be promoted.

In another embodiment, the aqueous liquid preparation of the present invention characteristically contains a water-soluble polymer to promote the intraocular penetration of the active ingredient.
Examples of the water-soluble polymer include xanthan gum, hyaluronic acid, polyethylene glycol, polyvinylpyrrolidone, hydroxypropylmethylcellulose, methylcellulose, hydroxyethylcellulose, alginic acid, polyglutamic acid, chitosan, carboxymethylcellulose, a salt thereof, carboxyvinyl polymer, polyvinyl alcohol and the like. Preferred is xanthan gum capable of affording appropriate viscosity of an aqueous liquid preparation.

For example, the average molecular weight of xanthan gum is generally 100,000 - 50,000,000, preferably 200,000 - 20,000,000, particularly preferably 1,000,000 - 10,000,000. As the xanthan gum, ECHO GUM series such as ECHO GUM T, ECHO GUM F and the like commercially available from Dainippon Sumitomo Pharma Co., Ltd., SAN-ACE series such as SAN-ACE NXG-S and the like commercially available from San-Ei Gen F.F.I., Inc., KETROL series such as KETROL CG, KETROL CG-T and the like commercially available from SANSHO Co., Ltd., and the like are used, with preference given to ECHO GUM T and KETROL CG-T.

The concentration of the water-soluble polymer (e.g., xanthan gum) in the aqueous liquid preparation of the present invention is generally 0.02 - 1.0 w/v%, preferably 0.1 - 0.5 w/v%.

The viscosity of the aqueous liquid preparation of the present invention containing a water-soluble polymer, as measured by type E rotational viscometer (10 rpm), is generally not less than 30 mPa·s. An aqueous liquid preparation having this range of viscosity is preferable since, for example, it is easy to handle during intraocular topical administration, and superior in the liquid preparation intraocular storage.

For example, when xanthan gum is used, an eye drop having its concentration within 1.0 w/v% is preferable since it maintains suitable static viscosity of the preparation, has low viscosity under the administration (dynamic) conditions such as instillation and the like, and is easy to handle. It is also preferable during production of an aqueous liquid preparation since the viscosity is appropriate, and sterilization by filtration can be performed under general conditions.

The aqueous liquid preparation of the present invention is administered orally or parenterally. Examples of the administration form include oral administration forms such as syrup and the like and parenteral administration forms such as external preparations (e.g., solution, suspension or emulsion injection, eye drop and the like). It is preferably used in the form of an ophthalmic topical administration, and particularly preferably used in the form of an eye drop.

A preparation having the aforementioned dosage form can be prepared by mixing the present compound with an additive necessary for formulating a preparation, such as typical carrier, excipient, binder, stabilizer and the like and by following a conventional method. For example, the present compound is mixed with a pharmaceutically acceptable carrier (e.g., excipient, binder, disintegrator, corrective, corrigent, emulsifier, diluent, solubilizer and the like) to give a pharmaceutical composition or a pharmaceutical preparation in the form of syrup, liquid preparation, emulsion, suspension, injection (e.g., liquid preparation, suspension and the like), eye drop and the like in the form suitable for oral or parenteral preparation.

When preparing a liquid preparation, an additive, such as sodium chloride, glucose, sorbitol, glycerol, propylene glycol, ethyl alcohol and the like, is used.
When preparing an injection, a sterile aqueous solution such as physiological saline, isotonic solution, oil and the like are used. Where necessary, a suitable suspending agent such as sodium carboxymethylcellulose, nonionic surfactant, solubilizer (e.g., benzyl benzoate and benzyl alcohol), and the like can be concurrently used.
Moreover, when an eye drop is prepared, an aqueous solution or suspension is used, which is particularly a sterile injectable aqueous solution. The eye drop can appropriately contain various additives such as buffer, stabilizer, wetting agent, emulsifier, suspending agent, surfactant, isotonicity agent, preservative and thickener.

The buffer may be, for example, phosphate buffer, borate buffer, citrate buffer, tartrate buffer, acetate buffer, amino acid and the like.
The stabilizer may be, for example, sodium edetate, citric acid and the like.
The wetting agent may be, for example, glycerol and the like.
The suspending agent may be, for example, hydroxypropylmethylcellulose, methylcellulose and the like.
The surfactant may be, for example, polysorbate 80, polyoxyethylene hydrogenated castor oil and the like.
The isotonicity agent may be, for example, saccharides such as sorbitol, glucose, mannitol and the like, polyhydric alcohols such as glycerol, propylene glycol and the like, salts such as sodium chloride and the like, and the like.

The preservative may be, for example, quaternary ammonium salt such as benzalkonium chloride, benzethonium chloride and the like, p-hydroxybenzoate such as methyl p-hydroxybenzoate, ethyl p-hydroxybenzoate and the like, benzyl alcohol, phenethyl alcohol, sorbic acid and salts thereof, thimerosal, chlorobutanol and the like.
The thickener may be, for example, hydroxyethylcellulose, hydroxypropylcellulose, methylcellulose, hydroxypropylmethylcellulose, carboxymethylcellulose, salts thereof and the like.

When in use as an eye drop, pH is adjusted generally to about 4 - 9, preferably about 6 - 8.5.

The aqueous liquid preparation of the present invention contains the active ingredient in a proportion of generally 0.0001 - 10 w/v%, preferably 0.001 - 1 w/v%, more preferably 0.01 - 0.1 w/v%, of the preparation. While the dose and administration frequency vary depending on symptom, age, body weight and administration form, when it is used as an eye drop for an adult, a preparation containing the present compound in a proportion of 0.0001 - 10 w/v%, preferably 0.001 - 1 w/v%, is administered several times a day, preferably 1 - 2 times a day, more preferably once a day, by several drops, preferably 1 - 3 drops, each time.

### Examples

The present invention is explained in detail in the following by referring to Examples and Experimental Example, which are not to be construed as limitative.

### Examples 1 - 3 and Comparative Example 1

### Preparation of eye drop

According to the formulation shown in Table 1, an eye drop containing a compound having a Rho kinase inhibitory action, (R)-(+)-N-(1H-pyrrolo[2,3-b]pyridin-4-yl)-4-(1-aminoethyl)benzamide·monohydrochloride (compound A), was prepared. The mixing ratio (molar ratio) of compound A and an ion-pairing reagent in Examples 1 - 3 was 1:2.

**Table 1**

| ingredient | Example 1 | Example 2 | Example 3 | Comparative Example 1 |
|---|---|---|---|---|
| compound A | 0.0565 g | 0.0565 g | 0.0565 g | 0.0565 g |
| sodium octanoate | 0.052 g | - | - | - |
| sodium sorbate | - | 0.047 g | - | - |
| sodium N-lauroyl-L-glutamate | - | - | 0.11 g | - |
| sodium dihydrogen phosphate | 0.1 g | 0.1 g | 0.1 g | 0.1 g |
| sodium chloride | 0.85 g | 0.85 g | 0.85 g | 0.85 g |
| benzalkonium chloride | 0.005 g | 0.005 g | 0.005 g | 0.005 g |
| sodium hydroxide/hydrogen chloride | e.q. | e.q. | e.q. | e.q. |
| sterile purified water | e.q. | e.q. | e.q. | e.q. |
| total amount | 100 ml | 100 ml | 100 ml | 100 ml |
| pH | 7.0 | 7.0 | 7.0 | 7.0 |

### Experimental Example 1

### Penetration test of compound A into the aqueous humor by addition of ion-pairing reagent

The eye drops of Examples 1 - 3 and Comparative Example 1 were each administered by instillation (50 µL, once, n=4) to the eyes of male white rabbits (body weight 2.1 - 2.7 kg). The rabbits were euthanized 1 hr after the instillation administration by the administration of an excess amount of pentobarbital sodium solution. The external segment of the eye was washed with saline, and aqueous humor was sampled with a syringe with a 27G injection needle. The recovered aqueous humor was filtered with a 0.45 µm chromato disc, and the filtrate was used as a sample solution. HPLC was performed under the following conditions, and the concentration of compound A was measured.
<HPLC conditions>
detector: ultraviolet absorptiometer (measurement wavelength: 248 nm)
column: YMC-Pack ODS A-302 (4.6 mm×15 cm, 5 µm)
column temperature: constant temperature near 40°C
mobile phase: mixed solution of methanol:0.02 mol/L sodium perchlorate (pH 2.5)=18:82
flow rate: 1.0 mL/min
injection volume: 50 µL
<Results>
The results are shown in Table 2.

**Table 2**

| group | concentration (ng/mL) of compound A in aqueous humor * |
|---|---|
| Comparative Example 1 | 23.7 ±14.7 |
| Example 1 | 44.0 ±5.7 |
| Example 2 | 46.6 ±23.3 |
| Example 3 | 49.1 ±47.0 |

| | |
|---|---|
| * Each value shows average ± standard deviation. | |

As shown in Table 2, the concentrations of compound A in the aqueous humor in the Example 1 administration group, Example 2 administration group and Example 3 administration group increased as compared to that in the Comparative Example 1 administration group. Therefrom it was clarified that addition of sodium octanoate, sodium sorbate or sodium N-lauroyl-L-glutamate, which are ion-pairing reagents, to an aqueous liquid preparation containing compound A promotes penetration of compound A into the aqueous humor.

### Example 4 and Comparative Example 2

### Preparation of eye drops

According to the formulations shown in Table 3, eye drops containing compound A were prepared.

**Table 3**

| ingredient | Example 4 | Comparative Example 2 |
|---|---|---|
| compound A | 0.0565 g | 0.0565 g |
| xanthan gum (ECHO GUM T: registered trade mark, Dainippon Sumitomo Pharma Co., Ltd.) | 0.2 g | - |
| sodium dihydrogen phosphate | 0.1 g | 0.1 g |
| sodium chloride | 0.85 g | 0.85 g |
| sodium hydroxide/hydrogen chloride | e.q. | e.q. |
| sterile purified water | e.q. | e.q. |
| total amount | 100 ml | 100 ml |
| pH | 7.0 | 7.0 |

### Experimental Example 2

### Intraocular tissue penetration test of compound A by addition of water-soluble polymer

The eye drops of Example 4 and Comparative Example 2 were each administered by instillation (50 µL, once, n=5) to the eyes of male white rabbits (body weight 2.2 - 2.6 kg). The rabbits were euthanized 1 hr or 2 hr after the instillation administration by the administration of an excess amount of pentobarbital sodium solution. The external segment of the eye was washed with saline, and aqueous humor and conjunctiva were sampled. Aqueous humor was sampled with a syringe with a 27G injection needle, methanol (10 µL) and the following internal standard solution (10 µL) were added to aqueous humor (100 µL), the mixture was dried under a nitrogen stream, dissolved in methanol (20 µL) and the following mobile phase solution A (180 µL) and used as an aqueous humor sample solution. The conjunctiva was taken, acetonitrile (5 mL) was added and the conjunctiva was chopped and the supernatant (4.5 mL) was separated. This was dried under a nitrogen stream, and dissolved in the following dilution solution (500 µL).
Methanol (10 µL) and internal standard solution (10 µL) were added to this solution (100 µL), and the mixture was dried under a nitrogen stream. The residue was dissolved in methanol (20 µL) and mobile phase solution A (180 µL), and used as a conjunctiva sample solution. HPLC was performed under the following conditions, and the concentration of compound A in each sample solution was measured.
<HPLC conditions >
detector :MS/MS
column: Capcell Pak UG-120 S-3 (2.0 mm×5 cm, 5 µm) guard column: Rheodyne Column Inlet Filter (3 mm ID frit) column temperature: 30°C mobile phase solution A: 0.1% aqueous formic acid solution of 10 mM ammonium formate
mobile phase solution B: acetonitrile
injection volume: 20 µL
dilution solution: mixed solution of methanol:physiological saline=1:9
internal standard solution: internal standard substance ((+)-trans-4-(1-aminoethyl)-1-(4-pyridylcarbamoyl)cyclohexanedihydrochloride·monohydrate), about 1.4 µg/mL methanol solution

**Table 4**

| MS/MS conditions: ionizing method: turbo ion spray, cation mode | | |
|---|---|---|
| substance | Q1 (m/z) | Q3 (m/z) |
| compound A | 281 | 264 |
| internal standard substance | 248 | 95 |

**Table 5**

| gradient conditions | | | |
|---|---|---|---|
| time (min) | flow rate (mL/min) | ratio of mobile phase solution A (v/v%) | ratio of mobile phase solution B (v/v%) |
| 0.0 | 0.25 | 100 | 0 |
| 7.0 | 0.25 | 80 | 20 |
| 7.0 | 0.25 | 100 | 0 |
| 15.0 | 0.25 | 100 | 0 |

### <Results>

The results are shown in Table 6.

**Table 6**

| group | concentration (ng/mL) of compound A in aqueous humor * | | concentration (ng/g) of compound A in conjunctiva * | |
|---|---|---|---|---|
| | 1 hr from instillation | 2 hr from instillation | 1 hr from instillation | 2 hr from instillation |
| Comparative Example 2 | 56.2±34.1 | 64.3±33.0 | 159.8±64.4 | 65.1±33.1 |
| Example 4 | 153.7±146.4 | 96.9±38.9 | 291.2±136.4 | 142.3±161.8 |

| | | | | |
|---|---|---|---|---|
| * Each value shows average ± standard deviation. | | | | |

As shown in Table 6, the concentration of compound A in the aqueous humor in the Example 4 administration group increased as compared to that in the Comparative Example 2 administration group both at 1 hr and 2 hr from the instillation. The concentration of compound A in the conjunctiva in the Example 4 administration group increased as compared to that in the Comparative Example 2 administration group both at 1 hr and 2 hr from the instillation. Therefrom it was clarified that addition of xanthan gum, which is a water-soluble polymer, to an aqueous liquid preparation containing compound A promotes penetrantion of compound A into the intraocular tissue.

### Experimental Example 3

### Intraocular tissue penetration test of compound A by addition of water-soluble polymer

The eye drops of Example 4 and Comparative Example 2 were subjected to a test in the same manner as in Experimental Example 2, and the concentration of compound A in aqueous humor and conjunctiva was measured at 6 hr and 12 hr from the instillation (n=3).

### <Results>

The results are shown in Table 7.

**Table 7**

| group | concentration (ng/mL) of compound A in aqueous humor * | | concentration (ng/g) of compound A in conjunctiva * | |
|---|---|---|---|---|
| | Comparative Example 2 administration group | Example 4 administration group | Comparative Example 2 administration group | Example 4 administration group |
| 6 hr later | 18.6 ± 3.8 | 41.5 ± 22.0 | 34.2 ± 35.2 | 61.6 ± 55.2 |
| 12 hr later | 2.7 ± 1.5 | 8.3 ±7.5 | 13.4 ± 20.2 | 15.9 ± 15.9 |

| | | | | |
|---|---|---|---|---|
| * Each value shows average ± standard deviation. | | | | |

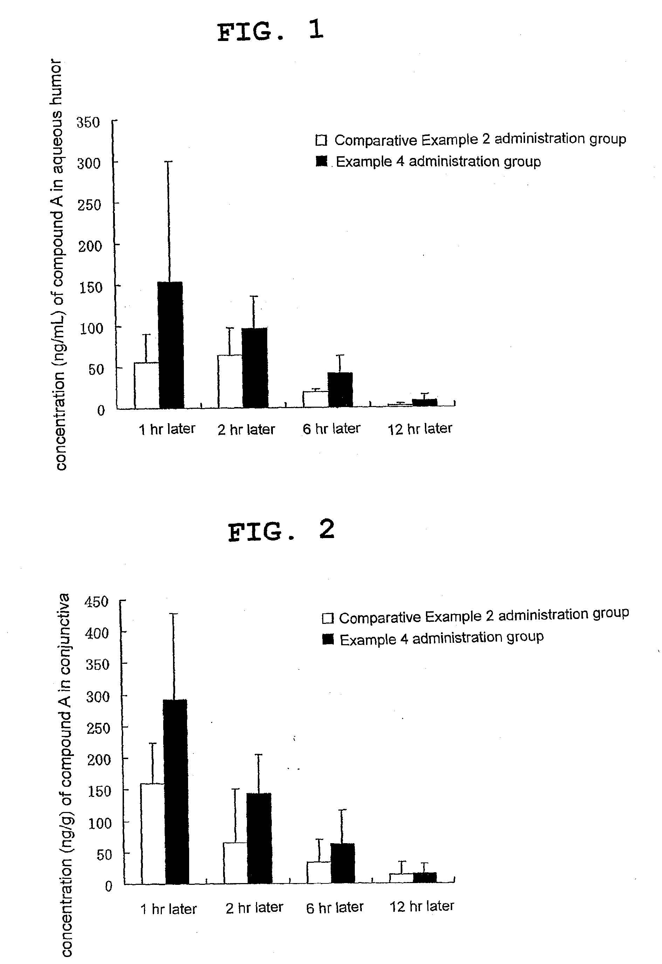

As shown in Table 7, the concentration of compound A in the aqueous humor in the Example 4 administration group increased 2 times or more that in the Comparative Example 2 administration group both at 6 hr and 12 hr from the instillation. The concentration of compound A in the conjunctiva in the Example 4 administration group increased about twice that in the Comparative Example 2 administration group at 6 hr from the instillation. Therefrom it was clarified that addition of xanthan gum, which is a water-soluble polymer, to a water-soluble liquid preparation containing compound A promotes penetration of compound A into the intraocular tissue, and the effect thereof is sustained for a long time.
After the instillation, the concentrations of compound A in the aqueous humor and the concentrations of compound A in the conjunctiva in the Experimental Example 2 and Experimental Example 3 described above are shown in Fig. 1 and Fig. 2, respectively.

### Industrial Applicability

According to the aqueous liquid preparation of the present invention, intraocular penetration of the present compound which is an active ingredient is promoted, a treatment-effective concentration of the active ingredient can be maintained for a long time in an intraocular tissue, and the administration frequency can be reduced. According to the present invention, a preparation superior in the action site reacheability of the active ingredient, effective use of the ingredient and reduction of administration frequency, as compared to known preparations containing the same concentration of the active ingredient, can be provided. According to the present invention, moreover, even a preparation containing a low concentration of the active ingredient can exhibit efficacy equivalent to that of known preparations, and extend the administration interval. Thus, a preparation less burdensome particularly to patients under long-term administration can be provided.
This application is based on a patent application No. 2006-209102 (filing date: July 31, 2006) filed in Japan, the contents of which are incorporated in full herein by this reference.

## Claims

1. An aqueous liquid preparation comprising an amide compound represented by the following formula (I) 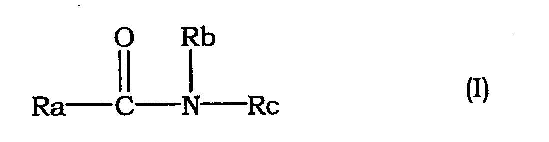 wherein
Ra is a group of the formula 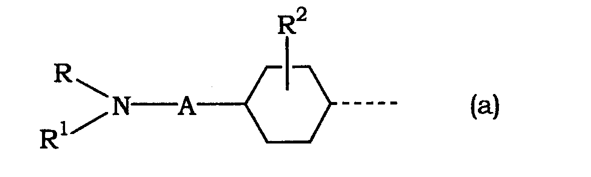 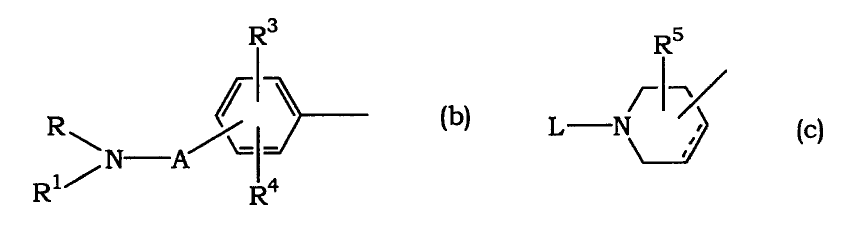
in the formulas (a) and (b),
R is hydrogen, alkyl, or cycloalkyl, cycloalkylalkyl, phenyl or aralkyl, which optionally has a substituent on the ring, or a group of the formula 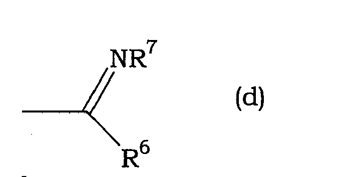
wherein R⁶ is hydrogen, alkyl or formula:-NR⁸R⁹ wherein R⁸ and R⁹ are the same or different and each is hydrogen, alkyl, aralkyl or phenyl, R⁷ is hydrogen, alkyl, aralkyl, phenyl, nitro or cyano, or R⁶ and R⁷ in combination show a group forming a heterocycle optionally further having, in the ring, oxygen atom, sulfur atom or optionally substituted nitrogen atom,
R¹ is hydrogen, alkyl, or cycloalkyl, cycloalkylalkyl, phenyl or aralkyl, which optionally has a substituent on the ring,
or R and R¹ in combination form, together with the adjacent nitrogen atom, a group forming a heterocycle optionally further having, in the ring, oxygen atom, sulfur atom or optionally substituted nitrogen atom,
R² is hydrogen or alkyl,
R³ and R⁴ A are the same or different and each is hydrogen, alkyl, aralkyl, halogen, nitro, amino, alkylamino, acylamino, hydroxy, alkoxy, aralkyloxy, cyano, acyl, mercapto, alkylthio, aralkylthio, carboxy, alkoxycarbonyl, carbamoyl, alkylcarbamoyl or azide, and is a group of the formula 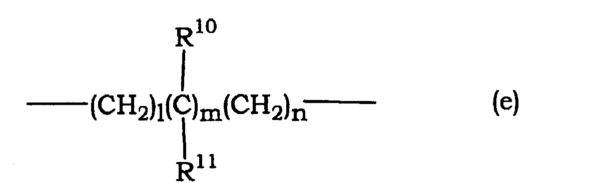 wherein R¹⁰ and R¹¹ are the same or different and each is hydrogen, alkyl, haloalkyl, aralkyl, hydroxyalkyl, carboxy or alkoxycarbonyl, or R¹⁰ and R¹¹ show a group which forms cycloalkyl in combination and 1, m and n are each 0 or an integer of 1-3,
in the formula (c),
L is hydrogen, alkyl, aminoalkyl, mono- or dialkylaminoalkyl, tetrahydrofurfuryl, carbamoylalkyl, phthalimidoalkyl, amidino or a group of the formula 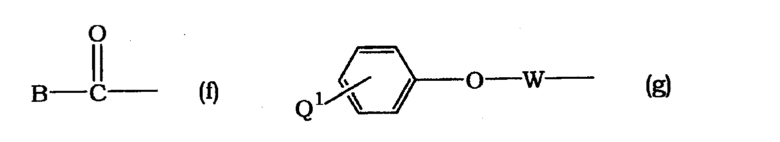 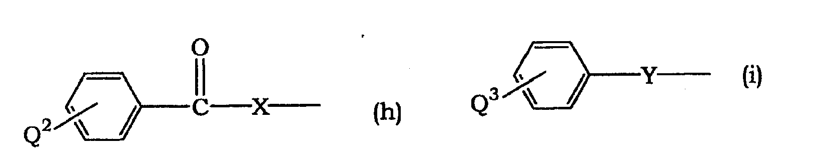 wherein B is hydrogen, alkyl, alkoxy, aralkyl, aralkyloxy, aminoalkyl, hydroxyalkyl, alkanoyloxyalkyl, alkoxycarbonylalkyl, α-aminobenzyl, furyl, pyridyl, phenyl, phenylamino, styryl or imidazopyridyl,
Q¹ is hydrogen, halogen, hydroxy, aralkyloxy or thienylmethyl,
W is alkylene,
Q² is hydrogen, halogen, hydroxy or aralkyloxy,
X is alkylene,
Q³ is hydrogen, halogen, hydroxy, alkoxy, nitro, amino, 2,3-dihydrofuryl or 5-methyl-3-oxo-
2,3,4,5-tetrahydropyridazin-6-yl;
and Y is a single bond, alkylene or alkenylene, and
in the formula (c),
a broken line is a single bond or a double bond, and
R⁵ is hydrogen, hydroxy, alkoxy, alkoxycarbonyloxy, alkanoyloxy or aralkyloxycarbonyloxy;
Rb is hydrogen, alkyl, aralkyl, aminoalkyl or mono- or dialkylaminoalkyl; and
Rc is an optionally substituted heterocycle containing nitrogen,
an isomer thereof and/or a pharmaceutically acceptable acid addition salt thereof, and an ion-pairing reagent or water-soluble polymer.

2. The aqueous liquid preparation of claim 1, wherein the ion-pairing reagent is sorbic acid, octanoic acid or N-lauroyl-L-glutamic acid or a pharmacologically acceptable salt thereof.

3. The aqueous liquid preparation of claim 1, wherein the water-soluble polymer is xanthan gum.

4. The aqueous liquid preparation of any one of claims 1 to 3,
wherein the compound represented by the formula (I) is (R)-(+)-N-(1H-pyrrolo[2,3-b]pyridin-4-yl)-4-(1-aminoethyl)benzamide·monohydrochloride.

5. The aqueous liquid preparation of any one of claims 1 to 4, which is an eye drop.

6. A method of promoting intraocular penetration of a compound represented by the formula (I) of claim 1, an isomer thereof and/or a pharmaceutically acceptable acid addition salt thereof, which comprises adding an ion-pairing reagent or a water-soluble polymer to an aqueous solution comprising the compound represented by the formula (I), or an isomer thereof and/or a pharmaceutically acceptable acid addition salt thereof.

7. A method for the prophylaxis or treatment of a disease selected from the group consisting of glaucoma, asthenopia and pseudomyopia caused by sustained abnormal tension of ciliary muscle, a visual functional disorder caused by damage or degeneration of retinal nerve or optic nerve, corneal sensitivity dysfunction caused by damage to corneal nerve and dry eye caused by corneal sensitivity dysfunction, which comprises administering an effective amount of the compound represented by the formula (I) of claim 1, an isomer thereof and/or a pharmaceutically acceptable acid addition salt thereof to a subject together with an ion-pairing reagent or a water-soluble polymer.

8. The method of claim 7, wherein the ion-pairing reagent is sorbic acid, octanoic acid or N-lauroyl-L-glutamic acid or a pharmacologically acceptable salt thereof.

9. The method of claim 7, wherein the water-soluble polymer is xanthan gum.

10. The method of any one of claims 7 to 9, wherein the compound represented by the formula (I) is (R)-(+)-N-(1H-pyrrolo[2,3-b]pyridin-4-yl)-4-(1-aminoethyl)benzamide· monohydrochloride.

11. The method of any one of claims 7 to 10, which relates to an eye drop.

12. Use of the compound represented by the formula (I) of claim 1, an isomer thereof and/or a pharmaceutically acceptable acid addition salt thereof, and an ion-pairing reagent or a water-soluble polymer for the production of an water-soluble liquid preparation for the prophylaxis or treatment of a disease selected from the group consisting of glaucoma, asthenopia and pseudomyopia caused by sustained abnormal tension of ciliary muscle, a visual functional disorder caused by damage or degeneration of retinal nerve or optic nerve, corneal sensitivity dysfunction caused by damage to corneal nerve and dry eye caused by corneal sensitivity dysfunction.

13. The use of claim 12, wherein the ion-pairing reagent is sorbic acid, octanoic acid or N-lauroyl-L-glutamic acid or a pharmacologically acceptable salt thereof.

14. The use of claim 12, wherein the water-soluble polymer is xanthan gum.

15. The use of any one of claims 12 to 14, wherein the compound represented by the formula (I) is (R)-(+)-N-(1H-pyrrolo[2,3-b]pyridin-4-yl)-4-(1-aminoethyl)benzamide· monohydrochloride.

16. The use of any one of claims 12 to 15, which relates to an eye drop.

17. A commercial package comprising the aqueous liquid preparation of any one of claims 1 to 5 and a written matter stating that the aqueous liquid preparation can or should be used for the prophylaxis or treatment of a disease selected from the group consisting of glaucoma, asthenopia and pseudomyopia caused by sustained abnormal tension of ciliary muscle, a visual functional disorder caused by damage or degeneration of retinal nerve or optic nerve, corneal sensitivity dysfunction caused by damage to corneal nerve and dry eye caused by corneal sensitivity dysfunction.
